# EUROPEAN PATENT SPECIFICATION

(11) **EP 2 731 640 B1**
(45) Date of publication and mention of the grant of the patent: **14.09.2016**
(21) Application number: 12740527.2
(22) Date of filing: 13.07.2012
(51) Int. Cl.: A61M 5/14, A61M 5/168

(54) **MONITORING SYSTEM FOR A MEDICAL LIQUID DISPENSING DEVICE**
ÜBERWACHUNGSSYSTEM FÜR EINE ABGABEVORRICHTUNG FÜR MEDIZINISCHE FLÜSSIGKEIT
SYSTÈME DE SURVEILLANCE POUR DISPOSITIF DE DISTRIBUTION DE LIQUIDE MÉDICAL

(30) Priority: 15.07.2011 EP 11174160
(43) Date of publication of application: 21.05.2014
(73) Proprietor: Spronken, Leon, 3600 Genk (BE)
(72) Inventor: SPRONKEN, Leon, 3600 Genk (BE); DAENEN, Michael, 3740 Bilzen (BE); THOELEN, Ronald, 3520 Zonhoven (BE); CLAES, Vincent, 3500 Hasselt (BE)
(74) Representative: Paemen, Liesbet R.J.
(86) International application number: PCT/EP2012/063752
(87) International publication number: WO 2013/010928

(56) References cited:
- WO-A1-94/01193
- WO-A1-2010/054314
- WO-A2-2008/008281
- CN-A- 102 028 990
- US-A- 5 651 775
- US-A- 6 106 727
- US-A1- 2006 064 053

## Description

### FIELD OF THE INVENTION

The present invention relates generally to the field of monitoring systems for medical infusion devices. More particularly, the present invention relates to monitoring systems for medical liquid dispensing devices which enable a healthcare provider to be alerted when a predetermined amount of fluid has been infused, or the infusion bag is nearly empty. In addition, the systems of the present invention allow to monitor and control the delivery of specific medication to a specific patient by a medical liquid dispensing device.

### BACKGROUND

The use of supply bags and bottles to introduce nutrient, therapeutic and medicinal fluids by intravenous, oral or nasal administration into the human body is well-known in the art. Typically, such systems have a container suspended above a patient by means of an bag stand or the like. The container is hung from the stand, and fluid flows from a spike inserted in the lower portion of the container through a delivery tube inserted (for example via an IV needle) into the patient, where it is infused into the patient's body.

To ensure a regular and uninterrupted flow of fluid to the patient, it is necessary to monitor the fluid level in the container so that it can be replaced when the fluid is low. Alternatively, the fluid in the container may be replenished. In either case, it is necessary to have an attendant continuously or periodically monitor the fluid level or to provide an automated monitoring system.

Furthermore, since there is an increasing complexity in the types of treatment and services available, hospitals and health care institutions must be able to provide these complex treatments and services while maintaining complete and detailed medical records for each patient. While in many hospitals and clinical laboratories, a bracelet device (e.g. wristband) having a patient's name printed thereon is permanently affixed to a patient upon admittance to the institution in order to identify the patient during his or her entire stay, opportunities still arise for patient identification errors.
Also, manually transferring information, such as the parameters for configuring an infusion device to dispense medication may result in errors that reduce the accuracy and/or effectiveness of drug administration and patient care.

Medical errors, such as where the wrong patient receives the wrong drug at the wrong time, in the wrong dosage or even where the wrong surgery is performed, are a significant problem for all health care facilities. Many prescription drugs and injections are identified merely by slips of paper on which the patient's name and identification number have been handwritten by a nurse or technician who is to administer the treatment. For a variety of reasons, such as the transfer of patients to different beds and errors in marking the slips of paper, the possibility arises that a patient may be given an incorrect treatment.

Devices which monitor the infusion of intravenous and irrigation solutions and provide a warning when such infusion has reached a predetermined limit are well known in the art.

One category of known devices makes use of a scale mechanism, such as a see-saw. When the amount of remaining fluid falls below a predetermined value, the see-saw tilts and actuates an electrical switch to trigger an alarm. Although the scale-based devices are simple, the springs used in the scales are quite sensitive to jolts or shocks that can occur, for example, if the patient accidentally strikes the device.

Another type of prior art device makes use of a clip or clamp together with elements that complete an electrical signal circuit as the reservoir bag collapses on emptying. However, the clamp can affect the rate of flow of the fluid and the alerting signal is only activated when the reservoir is empty.

Still other prior art devices use optical sensing equipment to sense the fluid level of the dripper and to monitor the fluid delivery system for occlusion or bubbles in the fluid. However, these devices have been found to be extremely complex and expensive.

All of these devices either monitor infusion rate or fluid empty condition.

Furthermore, various systems for monitoring and controlling patient care are known, including systems that use bar codes to identify patients and medications, or systems allowing the bedside entry of patient data. While these systems have advanced the art significantly, even more comprehensive systems could prove to be of greater value.

Accordingly, there is an urgent need for an integrated system allowing the monitoring of intravenous administration to different patients.

### SUMMARY OF THE INVENTION

The present invention provides medical liquid infusion monitoring systems which overcome one or more of the shortcomings of the prior art devices described above. The systems can be used to monitor any type of automated administration of fluid to a patient.

In one aspect, the present invention provides monitoring systems for monitoring the dispensing of a liquid from a medical liquid dispensing device for administration into a patient comprising:
- an identification unit for identifying a medical liquid container via a data storage medium comprising a product identifier,
- a weight monitoring unit for measuring the weight of the medical liquid container when liquid is dispensed therefrom for administration to said patient, and
- a processing unit for processing and/or storing data acquired from the identification unit and the weight monitoring unit, which processing unit is interconnected with the identification unit and the weight monitoring unit via a wireless or physical communications interface;
wherein the processing unit is configured to compare information concerning the identity of said patient, uploaded as a patient identifier, with information obtained from the product identifier.

The processing unit comprises a processor configured for processing data acquired from the identification unit and the weight monitoring unit. In particular embodiments, the processor is a microprocessor, which may be incorporated into the identification unit of the monitoring systems of the present invention. Alternatively, the processing unit or microprocessor may be integrated in the weight monitoring unit or is integrated in a separate device. In particular embodiments, the processing unit of the monitoring systems of the present invention is further connected to an external database server system.

The processing unit comprises a memory for storing data acquired from the identification unit and the weight monitoring unit. These data acquired from the identification unit and the weight monitoring unit include information concerning the identity and/or nature of the medical liquid container. The information concerning the identity of the medical liquid container at least includes information concerning the weight of the medical liquid container and optionally concerning the content of the medical liquid container. In particular embodiments the identification unit can further be used to identify the patient to be treated with the medical liquid via a data storage medium. In further particular embodiments, the weight monitoring unit of the monitoring systems of the present invention is or comprises at least one, i.e. one or more electronic weight sensors.

In particular embodiments, the identification unit of the monitoring systems of the present invention comprises a reader and the data storage medium is or comprises a machine-readable label.

In a further aspect, the present invention provides methods for monitoring the dispensing of a liquid from a medical liquid dispensing device for administration to a patient, which are based on:
- linking information on said patient uploaded as patient identifier with information concerning the medical liquid container obtained from the product identifier and/or
- linking weight information of the medical liquid container to a weight monitoring unit and/or on processing information of the medical liquid container and linking and comparing the information concerning the medical liquid container to information concerning said patient. More particularly the methods of the invention comprise one or more of the following steps of:
- identifying by means of an identification unit a medical liquid container via a data storage medium,
- comparing said identity of said medical liquid container with the information of the patient to be treated uploaded as a patient identifier by means of a processing unit,
- measuring the weight of the medical liquid container when liquid is dispensed therefrom by means of a weight monitoring unit, and
- linking the data acquired from the identification unit and the weight monitoring unit by means of said processing unit so as to monitor when the medical liquid container needs to be replaced and
- processing and/or storing data acquired from said identification unit and said weight monitoring unit by means of said processing unit, including comparing the patient information uploaded as patient identifier in said processing unit with information concerning the medical liquid container obtained from the product identifier.

In particular embodiments, the step of identifying a medical liquid container by means of an identification unit involves obtaining information via a data storage medium concerning the weight of the medical liquid container (and/or the liquid to be dispensed therefrom) and optionally concerning the content of the medical liquid container.

In particular embodiments, the information acquired from the weight monitoring unit comprises information on the weight decrease of the medical liquid container when liquid is dispensed therefrom.

In particular embodiments, the methods of the invention comprise the step of comparing the weight measured by the weight monitoring unit to a predetermined threshold value and optionally generating a warning signal when the measured weight corresponds to or is below the threshold value. Such a predetermined threshold value may in some embodiments be determined based on the information obtained from the data storage medium of the medical liquid container concerning the weight of the medical liquid container (and/or the liquid to be dispensed therefrom).

In particular embodiments, the methods of the present invention comprise the step of comparing the weight measured by the weight monitoring unit with an expected weight value based on the flow of the liquid dispensing device and optionally generating a warning signal when the measured weight is above or below the expected weight value by means of the processing unit.

In further particular embodiments, the methods of the present invention optionally comprise the step of generating a warning signal when a defect in the flow mechanism of the medical liquid container is detected based on the weight measured by the weight monitoring unit.

In particular embodiments, the identification unit is also used to identify the patient to be treated with the medical liquid via a second (or "patient-linked") data storage medium. In these embodiments, the methods of the invention may further comprise the step of comparing the information obtained from this data storage medium with the information obtained from the first (or "medical liquid container-linked") data storage medium.

In particular embodiments, the methods of the present invention additionally comprise the step of generating a warning signal by means of the processing unit when the information concerning the identity of the patient to be treated is incompatible with the information concerning the content of the medical liquid container.

The systems and methods of the present invention allow the detection of very low or no flow conditions. The system is not particularly sensitive to bumps or jolts, and remains accurate over time. The system is easy to install and use by medical attendants. Moreover, it can be set to differing amounts of liquid infusion, and optionally to calculate the rate of infusion.

In addition, the systems and methods of the invention provide an interrelated automated monitoring of delivery of therapeutic and other drugs to the patient, preventing administration of inappropriate or incorrect medication to a patient by checking the medication against a database comprising the patent's medical history. The systems also provide updated patient information at the bedside, e.g. when an additional drug is required, when a scheduled treatment is running behind schedule, and/or when the institution's accounting database needs to be updated.

Other objects and features of the present invention will become apparent from consideration of the following description taken in conjunction with the accompanying drawings.

### BRIEF DESCRIPTION OF THE DRAWING

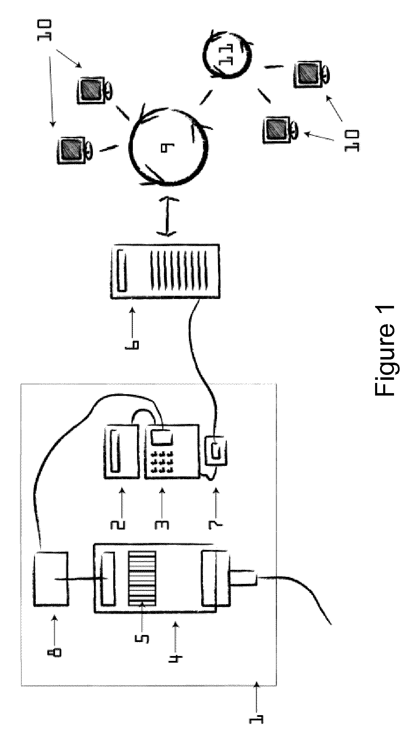

Figure 1 shows a schematic representation of a particular embodiment of a monitoring system (1) in accordance with the present invention.

An infusion delivery device is shown, comprising a medical liquid container (4) having a data storage medium (5) attached thereto. The monitoring system of the invention (1) includes an identification unit (2). The data storage medium (5) is attached to the medical liquid container (4).

The identification unit (2) is configured for transferring the uploaded information to the processing unit (3) and/or to the local network (9), or to a location remote from the provider facility, such as a database server (6). In the illustrated embodiment, the identification unit (2) is coupled via a processing unit (3) to a database server (6) via a wireless or physical communication interface (7), which database server may optionally be connected to a graphical user interface (10) (GUI), comprising for example one or more terminals, smart phones, ipads, etc. to an interface (7).

The weight monitoring unit (8) measures the weight of the medical liquid container (4) of the medical infusion delivery device when liquid is dispensed therefrom.

The processor comprised in the processing unit (3) is configured for processing data acquired from the identification unit (2) and the weight monitoring unit (8).

The local network (9) and the database server (6) may have access and/or be connected to a communications network (11), such as the internet. It will be understood to the skilled person that the connections illustrated as physical connections can in practice also be wireless connections.

### List of reference numerals used in Figure 1

The illustration represents a particular embodiment of the features concerned and the corresponding features are not to be interpreted as limited to this specific embodiment.
(1) Monitoring system
(2) Identification unit
(3) Processing unit
(4) Liquid container
(5) Data storage medium
(6) Database server
(7) Interface (cable/wireless)
(8) Weight monitoring unit
(9) Local network
(10) Graphical user interface (gui: terminal, ipad, ...)
(11) External communication (e.g. internet)

### DETAILED DESCRIPTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include embodiments "consisting of" the recited members, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

The present invention is generally concerned with monitoring systems for monitoring the dispensing of liquid from medical liquid dispensing devices for administration to patients. The systems are intended for use in any medical setting wherein a medical liquid container, such as an infusion bag with fluid, is to be infused into a patient and are aimed at reducing the need for visual checking by nurses or other medical assistants. The monitoring systems allow an off-site electronic follow-up of the nature and the progression of the liquid administration to the patient such that the need for on-site visual inspection is reduced and an automatic control of compatibility of liquid and patient can be ensured. Furthermore, the systems of the present invention allow improved patient care management by being capable of monitoring, controlling and tracking the administration of specific medication to a specific patient.

This is achieved by a combination of an identification unit, which identifies a medical liquid container and optionally the patient to be treated, a weight monitoring unit, which measures the weight of the medical liquid container when liquid is dispensed therefrom, and a processing unit, which processes and/or stores data that are acquired from the identification unit and the weight monitoring unit. The identification of the medical liquid container by the identification unit ensures that information about the weight of the liquid to be dispensed and optionally the nature of the liquid is registered in the system. The weight monitoring unit to which the medical liquid container is secured typically generates information on the weight of the medical liquid container during dispensing from the medical liquid container i.e. during administration to the patient. In the processing unit, prior to and/or during the coupling of the medical liquid container to the weight monitoring unit and dispensing liquid therefrom to the patient, information on the identity of the patient to be treated is compared with the information about the medical liquid container and/or, during the dispensing of the liquid from the medical liquid container the weight change of the medical liquid container is compared to the weight of the liquid to be dispensed, such that the flow can be monitored and information on when a particular amount of the liquid is dispensed can be monitored from a distance.

In a first aspect, the present invention provides monitoring systems for monitoring the dispensing of a liquid from a medical liquid dispensing device for administration to a patient comprising:
- an identification unit for identifying a medical liquid containers via a medical data storage medium comprising a product identifier and optionally for identifying a patient to be treated carrying a patient identifier,
- a weight monitoring unit for measuring the weight of the medical liquid container when liquid is dispensed therefrom for administration to the patient, and
- a processing unit for processing and/or storing data acquired from the identification unit and the weight monitoring unit, which processing unit is interconnected with the identification unit and the weight monitoring unit via a wireless or physical communications interface; wherein the processing unit is configured to compare information from the weight monitoring unit with information obtained from the product identifier and/or information concerning the identity of the patient to be treated, uploaded as a patient identifier, with information obtained from the product identifier.

The identification unit of the monitoring systems of the present invention is a device which is configured to upload a product identifier, e.g. a product-specific identification code, and optionally a patient identifier, e.g. a patient-specific identification code, from one or more data storage media.

The nature of the data storage medium is not critical to the present invention and different types of data storage media are known in the art. They include storage media which merely store information by way of a code or signal, such as a barcode or a magnetic strip, but also include more sophisticated devices which store information such as an electronic recording device, e.g., a "smart" chip. In particular embodiments, the data storage medium is a barcode which is provided on the medical liquid container (in case of a product identifier) or on the bed or wristband of the patient (in case of a patient identifier).

The data storage medium linked to the medical liquid container includes a product identifier identifying the medical liquid container. The product identifier, i.e. the product specific identification code acquired by the identification unit, is linked to particular information concerning the identity of the medical liquid container, which can be accessed and uploaded by the processing unit comprised in the medical liquid monitoring systems of the invention, as further described herein.

Apart from the information concerning the (initial) weight and content of the medical liquid container, the product identifier may be linked to further information such as, but not limited to the product type and/or treatment purpose, its manufacturer, lot number, and/or expiration date. For example, a serial number system may be used that provides an exemplary serial number A12B1C2, wherein "A1" identifies a specific manufacturer, "2B" identifies a specific product, and "1C2" identifies a specific lot. Alternatively, the product identifier may be uniquely associated with one or more entries in the memory of the processing unit (as further described herein) e.g. the memory of a database that may be accessed to obtain all types of information related to the medical product.

In addition, the product identifier may be linked to other information, such as dosage data, e.g., identifying an amount and/or concentration of the medical product within the delivery device or identifying a required frequency of administration. Other optional information that may be stored includes product manufacturing date, administration requirements, instructions for use, and/or product warnings, such as possible allergic reactions or adverse interactions of the product with other medical products.

In particular embodiments, information concerning the identity of the medical liquid container at least includes information concerning the weight and/or volume of the medical liquid container (and/or the liquid to be dispensed therefrom), i.e. information concerning the initial weight or volume of a full medical liquid container prior to use.
In further particular embodiments, information concerning the identity of the medical liquid container further includes information concerning the identity of the patient who is intended to receive the particular medical product. This can be of use e.g. when patient-specific medical liquid containers are prepared on a separate location. In these embodiments, the information on the patient to be treated obtained by the identification of the medical liquid container can be compared with the records of the patient or, where applicable on information obtained from the patient via the identification unit.

Indeed, in particular embodiments, the identification unit is configured to additionally obtain data from a data storage medium linked to the patient to be treated. The patient data storage medium stores a patient identifier uniquely associated with the individual patient. As indicated above, the nature of the data storage medium is not critical to the invention and can be a barcode, a magnetic strip, or an electronic recording device, e.g. a "smart" chip. Typically, the data storage medium is attached to a wristband or other device secured to the individual patient, or, less preferably to the bed of the patient. Preferably, the nature of the patient data storage medium is the same as the data storage medium used to identify the medical liquid container.

Typically, the identity of a particular patient to be treated is linked to a patient-specific code, i.e. a patient identifier. A patient identifier is associated with an individual patient, i.e. is associated with that patient in such a way that the patient identifier uniquely identifies the individual patient.

The use of patient data storage media to identify the patient further allows the system to monitor whether adequate medical liquid will be administered to the right patient. Indeed, to the patient data storage medium provides a link to information concerning the identity of the particular patient to be treated, which information can be accessed via the processing unit comprised in the medical liquid monitoring systems of the invention as further described herein. Prior to administration of the medical liquid, it can be checked whether the information corresponds to the information concerning the patient present in a database. For example, by means of the patient identifier, a patient record associated with an individual patient may be accessed to confirm whether the individual patient is intended to receive the medical product identified by the product identifier. Once it is confirmed that the individual patient is intended to receive the medical product, the medical product may be administered to the individual patient.

In particular embodiments, the data storage medium is a code and the patient specific information can be automatically uploaded from the memory of the identification unit and/or the processing unit, as further described herein. In these embodiments, the patient record and/or the patient identifier may be stored in a memory of the processing unit, as further described herein.

Additionally or alternatively, where the data storage medium is a more sophisticated device comprising a memory, the patient specific information may be uploaded directly from the patient(-specific) data storage medium via the identification unit of the monitoring systems of the present invention.

In particular embodiments, as detailed above, information on the patient to be treated may be uploaded onto the data storage medium of the medical liquid container when the container is filled by the manufacturer.

Before starting with the dispensing of the medical liquid, i.e. before administering the medical liquid to the patient, the information of the medical liquid container can be checked with the information of the patient to be treated. For this purpose, the patient identifier may be uploaded from the patient data storage medium and the patient identifier may be compared with information obtained from the data storage medium of the medical liquid container to confirm that the medical liquid can be administered to the patient concerned. Alternatively, the patient data may be obtained from a database or may be stored in the processing unit.

In particular embodiments, the data storage medium for the patient identifier is a patient specific machine readable label which is provided on a wristband or other device that may be secured to a patient, or on a patient chart or other object kept in close proximity to the patient, e.g. on or adjacent to the patient's bed, wheelchair, and the like.

In particular embodiments, information on the medical liquid container is compared to the patient identifier and/or patient data accessible by or present in the memory of the processing unit as described above.

In particular embodiments, patient records may be stored in another device coupled to the monitoring system of the invention via a local network. The patient information may be retrieved therefrom and compared to information the product identifier uploaded by the identification unit to confirm whether the individual patient is intended to receive the medical liquid.

In particular embodiments, a medical liquid container is provided with a data storage medium attached thereto. A medical product is loaded into the liquid container by the manufacturer thereof, and a product identifier is downloaded onto the data storage medium, the product identifier identifying the medical product loaded into the liquid container.

The identification unit of the medical monitoring systems according to the present invention is typically a reader in the form of an electronic device, such as a scanner, which is configured for uploading information from a data storage medium, and for transferring the uploaded information to the processing unit. Generally, the reader may include a sensor and a display. Optionally, the reader may also include one or more other user interfaces, such as controls such as a keypad, a mouse, and the like.

Accordingly, in particular embodiments, the identification unit comprises a graphical user interface comprising for example a terminal, smart phone, ipad, etc..

As will be detailed below the identification unit is linked to a processing unit which may or may not be physically incorporated in the identification unit. Moreover, the identification unit may optionally be coupled to other components of a local network, e.g. to a local computer e.g. by an interface. The interface may be a cable directly coupling the reader to the local network or may be a wireless communications device, such as a short-range transmitter/receiver, a modem, and the like.

In particular embodiments, the reader is a stand-alone device, e.g. whereby the processing unit with a memory capable of performing any or all of the tasks described herein internally incorporated therein.

The identification unit, such as for example a reader, is configured for uploading information from a data storage medium, such as for example a product-specific machine-readable label. In particular embodiments, the identification unit is physically connected to an infusion delivery device, e.g. to the vertical bar of the infusion device, such that it allows easy access of the identification unit to the product identifier and optionally the patient identifier by the user (typically in standing position).

A communications interface may be coupled to the identification unit for acquiring the product-specific and patient-specific data. An external memory may be provided in a server or other provider computer within the local network for storing the product and patient data in a patient tracking file and may optionally be connected to a database. The weight and/or content of the medical container as well as the identity of the patient who is to receive the treatment can be further checked. In addition, other information concerning the medical product may be verified using the identification unit before delivering the medical product. For example, drug interaction data may be accessed from a product database related to the medical product identified by the product identifier. A patient history file of the individual patient may be accessed to confirm whether the medical product is interaction-free from other medical products already received by the individual patient. In particular embodiments, these verifications are performed automatically by the identification unit and/or by another computing device coupled to the identification unit, e.g., within a local network or via a communications network. Thus, in particular embodiments, as will be detailed below, the processing unit is connected to an external database server system. This external database may comprise product data and/or patient data.

In certain particular embodiments, the database may contain or may be used to store information on the different medicaments available in the institution for administration via a medical liquid infusion system. More particularly, these data can be introduced into the database using product identifiers, e.g. when the medical liquid containers are prepared. For instance, the product identifier may be uploaded into a local network at a provider location, e.g., using an identification unit as described herein. Product data may then be acquired from a processing unit, e.g. comprising a product database stored on a computer at a remote location from the local network, the product data being identified by the product identifier.

Similarly the database may contain or may be used to store information on the patient to be treated (i.e. to which the medical liquid is to be administered). For instance, it may be of interest to register administration date data relating to the infusion of the medical liquid into a patient data file at the time of administering the medical product to the individual patient. The patient data file is preferably stored on the local network, the patient data file preferably being linked to a patient identifier identifying the individual patient; the information relating to the medical fluid and the administration thereof may be added to the patient data file.

In addition, information identifying the individual provider or the medical staff responsible administering the medical product may also be recorded in a medical data file, thereby identifying responsible individuals. Indeed, in particular embodiments, the identification unit is configured to additionally obtain data from a data storage medium linked to the individual or medical staff responsible administering the medical product. In these particular embodiments, a data storage medium stores an identifier uniquely associated with the individual of the medical staff. The nature of the data storage medium is not critical to the invention and can be a barcode, a magnetic strip, or an electronic recording device, e.g. a "smart" chip. Typically, the data storage medium is attached to a wristband or other device secured to the individual of the medical staff who is responsible for administering the medical product and/or directly administers the product and/or is responsible for the patient. Preferably, the nature of the data storage medium is the same as the data storage medium used to identify the medical liquid container and/or the data storage medium used to identify the patient to be treated.

In particular embodiments the monitoring systems for medical liquid dispensing devices according to the present invention comprise more than one and up to multiple identification units. Indeed, it can be envisaged that where the system is used to monitor more than one medical liquid infusion dispensing device, each of the devices is provided with an identification unit for easy access to the product identifiers on the medical liquid container and optionally the patient identifier (present on or near the patient). In addition, a separate identification unit may be used to introduce information into the system relating to the production/availability of the medical liquid containers (i.e. at the production and/or storage facility) and/or the patients and/or the medical staff.

The monitoring systems for medical liquid dispensing devices according to the present invention further comprise a weight monitoring unit for measuring the weight of a medical liquid container when liquid is dispensed therefrom.

The weight monitoring unit consists of a device, to which the medical liquid container is attached and which measures the weight of the medical liquid container during the infusion process, i.e. during emptying of the container. Typically, the weight monitoring system is such that the medical liquid container suspends from the device and the weight is measured. In particular embodiments of the present invention, the weight of the medical liquid container is measured by using one or more load cells. Additionally or alternatively springs can be used.

In particular embodiments of the systems of the present invention, the weight of the medical liquid container is periodically measured and sent to the processing unit, as further described herein, in order to detect when the container is nearly empty, when there is a disruption in fluid-flow, or when a preselected amount of fluid loss has been reached.

In particular embodiments, the weight monitoring unit measures absolute weight values of the medical liquid container over time. In other particular embodiments, the weight monitoring unit measures the weight decrease of the medical container and thus measures relative differences between the absolute weights of the medical container over time.

The weight monitoring unit comprised in the medical liquid monitoring systems of the present invention may comprise one or more electronic weight sensing mechanisms or one or more electronic position sensors, such as for instance one or more transducers, load cells, or a Hall-Effect sensors, or any suitable combination thereof. In particular embodiments of the invention, the weight monitoring unit comprises one or more load cells.

Accordingly, in certain particular embodiments of the invention, where the medical liquid monitoring systems are used for monitoring medical liquid dispensing devices comprising only one medical liquid container, the weight monitoring unit comprises one weight sensing mechanism or one electronic weight sensor for measuring the weight of the medical liquid container when liquid is dispensed therefrom.

In certain embodiments, the one or more weight sensors are low cost devices based on a force-sensitive resistor; in such embodiments, an innovative hanging mechanism causes pressure on the force sensitive resistor, which force is directly related to the weight of the medical liquid container.

The weight monitoring unit in the systems of the present invention is linked to a processing unit, such that the information on the weight change of the medical liquid container (obtained either directly from the weight monitoring unit or by comparing subsequent weight data transmitted by the weight monitoring unit) can be compared with information obtained from the identification unit to determine when the medical liquid container is (almost) empty and needs to be replaced. This connection can be a physical connection or a wireless connection. In particular embodiments, as will be detailed below, the information on the weight change during administration to the patient is used to determine irregularities in the flow of the delivery system.

In particular embodiments of the invention, the medical liquid monitoring system of the invention is used for monitoring more than one medical liquid dispensing device and/or one medical liquid dispensing device comprising multiple, i.e. two or more, medical liquid containers. In these embodiments, the weight monitoring unit comprises multiple, i.e. two or more, weight sensing mechanisms or two or more electronic weight sensors, whereby each weight sensing mechanism or each weight sensor is used for measuring the weight of one particular medical liquid container when liquid is dispensed therefrom. In these particular embodiments, each weight sensing mechanism or weight sensor is predetermined, i.e. pre-programmed via the processing unit, to measure the weight of a certain particular medical liquid container. Determining which weight sensing mechanism or which weight sensor is pre-programmed to measure the weight of a certain particular medical liquid container, can be done by means of identifying the medical liquid container using the identification unit. Indeed, prior to hanging the medical container to one of the multiple stands of the medical liquid dispensing device(s), the responsible of the medical staff can identify the medical liquid container via a medical data storage medium using the identification unit of the medical monitoring systems of the invention. By identifying the medical container, information is exchanged with the processing unit, whereby it can be determined using any suitable method or procedure, to which stand (and thus to which weight sensing mechanism or weight sensor coupled thereto), the medical liquid container can be attached or hung.
The identification of which weight sensing mechanisms are pre-programmed to measure the weight of which medical containers can for example include the use of visual or audible indication systems, or procedures whereby the medical staff identifies the correct weight sensor for a particular container using an external database server system, which is connected to the processing unit of the monitoring systems of the invention.

The monitoring systems for medical liquid dispensing devices according to the present invention further comprise a processing unit. As detailed above, the processing unit is interconnected with the identification unit (as described herein above) and the weight monitoring unit (as described herein above) via a wireless or physical communications interface.

The processing unit can be incorporated into the identification unit, i.e. be physically present within the housing of the identification unit itself, e.g. in the form of a microprocessor, or can be coupled to the identification unit via an external network, comprising for example a database server system. Alternatively, the processing unit can be physically integrated into the weight monitoring unit or can be part of a separate device.

The processing unit comprises a processor configured for processing data acquired from the identification unit and the weight monitoring unit. In particular embodiments, the processing unit is configured for comparing the weight value of the medical liquid envisaged for administration as determined by the product identifier (acquired by the identification unit) with the change in the measured weight values obtained from the weight monitoring unit. In this way, the processing unit detects when the total amount for administration has been reached, e.g. when the medical container is nearly empty and/or when a preselected amount of fluid has been administered.

As detailed above, in particular embodiments, the processing unit, by comparing subsequent values obtained from the weight monitoring unit during dispensing of the fluid from the medical liquid container for administration of the medical liquid to the patient, identifies when there is a disruption in fluid-flow.

In certain particular embodiments, the processing unit is configured to compare data from the product identifier with data of the patient to be treated. The data of the patient to be treated can be introduced to the microprocessor either from an external database. Optionally, this information is introduced into the processing unit by uploading patient identifiers. The patient identifier corresponds to a particular patient bed in the hospital. In particular embodiments, the patient identifier can be uploaded by means of the identification unit. Patient-specific information linked to a patient identifier can be compared by the processing unit to the information present in a database and/or to the information obtained from the product identifier.

Accordingly, in these embodiments the processing unit is configured to compare the information from the patient identifier to the information obtained from the product identifier (and optionally in an internal database) in order to determine whether the patient information and the product information match.

Generally, with the monitoring systems of the present invention it can be determined, based on the processing of the data obtained from the identification unit and the weight monitoring unit, whether or not and at which rate the infusion liquid may be dispensed. For instance, when it is determined, after the processing of the data by the processing unit, that the data of the product identifier and the data of the patient identifier are incompatible or that the weight value measured by the weight monitoring unit has reached a certain threshold value, one or more warning signals or alarm conditions will be triggered and displayed on one or more (remote and/or central) monitoring screens. Such monitoring screens may be video displays attached to the nursing CPU and/or attached to other (e.g. bedside) processing units. Indeed, the status of each patient's infusion can be for instance represented on a video display at the nursing station.

In particular embodiments of the monitoring systems of the present invention, when such an alert occurs, a box representing the patient's room may flash to attract attention to the alert. Displaying the alarm condition in this manner allows a nurse to quickly and easily identify the patient from the nursing station and take appropriate action (by for instance preventing the medicament from being dispensed from the container or by (manually) adjusting the infusion rate) to address the condition causing the alarm.

In certain other particular embodiments, the monitoring systems of the present invention may be further configured such that the processing unit can be programmed to send signals/instructions to the liquid dispensing device. In these particular embodiments, when it is determined, after the processing of the data by the processing unit, that the data of the product identifier and the data of the patient identifier do not match, the processing unit is configured so as to communicate with the dispensing device by sending instructions that the infusion liquid comprising the medicament either is not to be dispensed from the container or is to be dispensed at an adjusted flow rate. Also in these particular embodiments, when it is determined by the processing unit that the weight value has reached a certain threshold value, instructions may be sent to the dispensing device to stop dispensing. Similarly in these particular embodiments, if it is established by the processing unit that for a certain patient a certain dosage regimen is required, instructions may be sent to the dispensing device that the dispensing of the liquid is to be performed in accordance with that particular dosage regimen.

An output device, e.g. a display on the reader, a speaker, and/or a printer, may be provided such as on the identification unit, for indicating the outcome of the processing unit, e.g. whether the patient identifier and the intended patient information match, thereby confirming whether the individual patient is intended to receive the medical product. Additionally or alternatively, all or part of the information can be transmitted to a central processing unit (CPU) for example at the nursing station and can be displayed centrally on one or more monitors.

Consequently, a nurse at the nursing station can access the status for a certain infusion dispensing device wherein the display connected to the CPU then displays information regarding the status of the (different) infusion(s) being performed at that time. For example, information can include the name of the drug being infused, the patient's name, the scheduled start, the actual start of infusion, the scheduled end of infusion, the projected end of infusion, the amount of drug infused, the amount of drug remaining to be infused and any alert or discrepancy conditions that may need attention by the nurse.

After evaluating the status of the infusion displayed on either the monitor at the CPU or on the video display (i.e. a monitor) at the bedside processing unit, the infusion regimen may be adjusted accordingly, in particular by the appropriate action of a nurse, care-giver or medical assistant or technician who for instance prevents the medicament from being dispensed from the container or (manually) adjusts the infusion rate or dosage regimen.

In particular embodiments, the processing unit comprises a memory for storing the information concerning the product that is linked to each particular product identifier, i.e. information concerning the (initial) weight, the content of the medical liquid container and, where applicable, the identity of the patient to be treated. In particular embodiments, the memory of the processing unit comprises patient data including information on the medical liquid to be administered.

As indicated above, in particular embodiments, the processing unit is present as a microprocessor. In certain particular embodiments, this microprocessor is integrated in the identification unit. Alternatively, this microprocessor may be integrated in the weight monitoring unit. In further particular embodiments, the processing unit is linked to a database server system. The system may include a computer at a location remote from the local network, the computer communicating with the local network via a communications network. The computer may include a medical product database, the computer configured for including at least a portion of the tracking file in the medical product database. For example, the server or other provider computer may periodically communicate at least a portion of patient data and product data in tracking files in the memory of the server or provider computer to a computer. In particular embodiments, the server or provider computer is configured for communicating at least a portion of patient data, e.g. demographic data of patients receiving respective medical products, to a computer while excluding personal information capable of identifying respective patients. In all embodiments, the identification unit is coupled to a processing unit via a communications interface, such as via a network.

As indicated above, the monitoring systems of the present invention may include one or more display units or monitors. Indeed, selective portions or all of the information acquired by the processor comprised in the processing unit may be displayed on a display, which may be any known output device, e.g., an LCD, LED's, a speaker, and/or a printer. The display may also provide visual and/or audio indications when the processor has performed one or more comparisons or confirmations, such as those tasks described above.

A memory, processor, and/or output device may be part of the processing unit and/or may be coupled to the identification unit via a computer network, e.g. a local area network.

As indicated above, the monitoring systems of the present invention may include one or more computers at a location remote from the local network, the computers communicating with the local network via a communications network. The computers may include a medical product database, and may be configured for including at least a portion of the tracking file in the medical product database. The server or other provider computer may periodically communicate at least a portion of patient data and product data in tracking files in the memory of the server or provider computer to the remote computers. Preferably, the server or provider computer is configured for communicating at least a portion of patient data, e.g. demographic data of patients receiving respective medical products, to a computer while excluding personal information capable of identifying respective patients.
Typically, in the monitoring systems of the invention, the data obtained from the identification unit as well as the data obtained from the weight monitoring unit are gathered in a central database, which database is configured to communicate with one or more devices that are able to trigger, send and/or display warning signals or alarm conditions on one or more (remote and/or central) monitoring screens. Such monitoring screens may be video displays attached to the nursing CPU and/or attached to other (e.g. bedside) processing units.

Nevertheless, in certain specific embodiments, the systems of the present invention may comprise a number of processing units having a variety of input and output devices for receiving patient data and for generating or displaying reports. A system of software programs operates on the processing units to record, process, and produce reports from a database whose data is representative of the care a patient receives in the institution. The processing units are connected together, along with at least one dedicated file server, to form a network. Patient data can be inputted by users of the personal computers, and can be stored in a data storage device connected to the file server.

Accordingly, in certain specific but non-limiting embodiments, the monitoring systems of the present invention include a computer, a central processing unit including a video display (i.e. a monitor) and printer and remote, such as for example bedside, processing units connected to one or more medical liquid dispensing devices for administration to (a) patient(s), one or more scanners (e.g. barcode readers) for reading barcode labels either affixed to the patient(s) (by means of an identification bracelet or wristband) and on the medical liquid container(s). In particular embodiments, the remote processing units may be integrated into the scanning device. In operation, this system can verify whether the right medication is being dispensed to the right patient in the right dosage via the right delivery route at the right time by comparing the information of the product identifier and the weight monitoring unit to that database information relating to the patient, the patient's condition, and the course of treatment prescribed to treat the patient's illness.

In further embodiments, the monitoring systems of the present invention comprise an identification unit that is passive in nature, i.e. the unit operates to automatically detect and identify an individual, such as a patient and/or caregiver without any particular action being required on the part of the individual patient. In further embodiments, an RF transponder is the identification unit, which is mounted at a patient's room or treatment area and automatically detects an identification device, such as a wrist band, on the individual to identify the individual.

In a further aspect, the present invention provides methods for monitoring a medical liquid dispensing device, more particularly using a system which ensures a link between the weight of the medical liquid to be administered and the actual change in weight of the medical liquid container during administration. Typically, methods for monitoring are provided comprising the steps of:
- identifying by means of an identification unit a medical liquid container via a data storage medium comprising a product identifier,
- measuring the weight of the medical liquid container when liquid is dispensed therefrom by means of a weight monitoring unit, and
- processing and/or storing data acquired from the identification unit and the weight monitoring unit by means of a processing unit such as to compare the weight change detected by the weight monitoring unit with the envisaged weight change for administration of the medical liquid and/or to compare the patient information uploaded as patient identifier in said processing unit with information concerning the medical liquid container obtained from the product identifier.

In particular embodiments, the step of identifying by means of an identification unit a medical liquid container via a data storage medium comprises obtaining information concerning the weight of the medical liquid container (e.g. information concerning the initial weight or volume of a full medical liquid container prior to use and/or information on the emptied medical liquid container after use, or information on the weight of the liquid to be administered) and optionally concerning the content of the medical liquid container.

Before administering the medical liquid to the patient to be treated, the medical liquid contained in the medical container may be identified by means of a product identifier. The product identifier may be uploaded from the data storage medium by means of an identification unit. In particular embodiments, the identification unit can upload a product identifier from a machine-readable label present on the medical liquid container. i.e. information concerning the initial weight or volume of a full medical liquid container prior to use.

In further particular embodiments, information concerning the identity of the medical liquid container further includes information concerning the content of the medical container, i.e. information concerning the nature and/or identity of the medical product comprised in the medical liquid container. In certain further particular embodiments, this information concerning the content of the medical container can be coupled, via the processing unit of the monitoring systems as further described herein, to the identity of the patient who is intended to receive the particular medical liquid.

In particular embodiments, the step of measuring the weight of a medical liquid container comprises measuring the weight decrease of the medical liquid container (when liquid is being dispensed therefrom) by means of a weight monitoring unit.

Generally, with the monitoring systems of the present invention it can be determined, based on the processing of the data obtained from the identification unit and the weight monitoring unit, whether or not and at which rate the infusion liquid may be dispensed. For instance, when it is determined, after the processing of the data by the processing unit, that the data of the product identifier and the data of the patient identifier are incompatible or that the weight value measured by the weight monitoring unit has reached a certain threshold value, one or more warning signals or alarm conditions will be triggered and displayed on one or more (remote and/or central) monitoring screens. Such monitoring screens may be video displays attached to the nursing CPU and/or attached to other (e.g. bedside) processing units. Indeed, the status of each patient's infusion can be for instance represented on a video display at the nursing station.

Thus, the methods of the present invention comprise the step of processing and/or storing data acquired from the identification unit and the weight monitoring unit by means of a processing unit, including comparing the patient information uploaded as patient identifier in the processing unit with information concerning the medical liquid container obtained from the product identifier.

In particular embodiments, the methods of the invention further comprise the step of comparing the weight measured by the weight monitoring unit to a predetermined threshold value and optionally generating a warning signal when the measured weight corresponds to or is below the threshold value by means of the processing unit. Such a predetermined threshold value may in some embodiments be determined based on the information obtained concerning the weight of the medical liquid container. In particular embodiments, the methods of the present invention further comprise the step of comparing the weight measured by the weight monitoring unit with an expected weight value based on the flow of the liquid dispensing device and optionally generating a warning signal when the measured weight is above or below the expected weight value by means of the processing unit.

In further particular embodiments, the methods of the present invention optionally comprise the step of generating a warning signal when a defect in the flow mechanism of the medical liquid container is detected by the processing unit based on the weight measured by the weight monitoring unit.

In still further particular embodiments, the methods of the present invention additionally comprise the step of generating a warning signal by means of the processing unit when the information concerning the identity of the patient to be treated is incompatible with the information concerning the content of the medical liquid container.

The systems of the present invention may be programmed or configured so as to immediately display warning signals or alarm conditions on remote monitoring screens, such as the video display (i.e. monitor) attached to the nursing CPU, as the alarm occurs. For example, the status of each patient's infusion can be represented on a video display at the nursing station. When an alert occurs, a box representing the patient's room may flash to attract attention to the alert. Displaying the alarm condition in this manner allows a nurse to quickly and easily identify the patient from the nursing station and take appropriate action to address the condition causing the alarm.

In particular embodiments, the data storage medium of the medical liquid container is a machine-readable label which is prepared, i.e. loaded with data prior to the use of the medical liquid container. In particular embodiments, a manufacturer or filler of a medical liquid container may download or otherwise provide information to one or more machine readable labels. For example, upon filling individual medical liquid infusion containers, bags or bottles, an electronic reader/writer may be used initially to transfer a product identifier and/or product data to the machine readable labels attached to respective infusion containers. If necessary or desired, the data may be read and/or supplemented during one or more steps of manufacturing, filling, or assembly. For barcode stickers, a bar code sticker printer may be used to generate initial bar code stickers, and/or to read existing bar code stickers and print new ones with new or supplemented data. The manufacturer may maintain a file in its computer system of all or part of the data downloaded to respective machine-readable labels and/or may transfer the data to a computer. The medical liquid infusion containers, including respective machine readable labels with data thereon, may be shipped to one or more provider locations for administration to respective patients.

In certain particular embodiments, the methods of the present invention include the step of comparing the patient information uploaded as patient identifier in the processing unit of the monitoring systems with information concerning the medical liquid container obtained from the product identifier in order to determine whether the patient identifier and the product information match. Where a processing unit is incorporated in the identification unit, an output device, e.g. a display on the reader, a speaker, and/or a printer, may be provided for indicating whether the patient identifier and the intended patient information match, thereby confirming whether the individual patient is intended to receive the medical product. Additionally or alternatively, all or part of the information is transmitted to a central processing unit (CPU) for example at the nursing station and is displayed centrally on one or more monitors.

Therefore, by applying the methods of the present invention, it can be accurately determined and verified whether the right medication is being dispensed to the right patient in the right dosage via the right delivery route at the right time by maintaining a database of information relating to the patient, the patient's condition, and the course of treatment prescribed to treat the patient's illness.

In particular embodiments of the methods of the present invention, a patient entering a health care institution is provided with a patient identifier, i.e. wristband necklace, ankle band or other identifier provided with a computer-readable label, that is affixed to the patient in a manner so that the patient can be identified even if the patient is unconscious or otherwise unresponsive. The patient identifier represents the name of the patient and other information that the institute has determined is important and also includes a computer-readable label, such as a barcode. The information printed upon the band, such as name, age, allergies or other vital information is encoded into the label or barcode.

After the patient is admitted and assigned to a bed within the institution, the patient is typically evaluated by a physician and a course of treatment is prescribed. The physician prescribes the course of treatment by preparing an order, which may request a series of laboratory tests or administration of a particular medication to the patient. The physician typically prepares the order by filling in a form or writing the order on a slip of paper to be entered into the hospital's system for providing care.

If the order is for administration of a particular medication regimen, the order will be will be transmitted, processed and prepared in the institution's pharmacy.

In certain particular embodiments, upon packaging the medication, a copy of the order, the patient's name, the drug name, and/or the appropriate treatment parameters may be represented on a label, which is fixed to the drug container.

According to one embodiment of the present invention, this information is represented for example by a barcode, which is printed on a label. The medication order and its information is made available by the hospital's pharmacy information system and is stored by the file server.

In this specific embodiment, the medication is then delivered to the appropriate unit for administering to the patient. A nurse or technician carries the drug container to the appropriate patient. In accordance with one embodiment of the present invention, the nurse or technician reads the barcode on the patient identifier (e.g. bracelet or wrist band) using a scanner or barcode reader connected to the bedside processing unit. Then, the product identifier, e.g. barcode on the label affixed to the drug container, is read. Additionally, a record of the identity of the caregiver dispensing the medication may be obtained by reading the barcode printed on an identity badge typically worn by institution personnel.

In the present specific embodiment, the data obtained are then processed by the processing unit, which records the therapeutic regimen information in the patient's history records, and verifies that the right medication is being given to the right patient in the right dose by the right route and at the right time. If a discrepancy is detected between the barcoded information printed on the patient bracelet and the barcoded information on the label affixed to the medication container, an alert is triggered and (the appropriate information) is displayed on a video display attached to either the central processing unit (CPU) (e.g. at the nursing station) and/or at one or more remote processing units, such as for example the bedside processing unit, i.e. located near to the infusion device of a (each) patient. The nurse or technician then corrects the discrepancy or overrides it, depending on the particular circumstances.

While particular embodiments of the present invention have been described above, alternative configurations are possible, where for example clinical devices are connected by means of appropriate interfaces and cabling to a central "bedside data concentrator", which would typically be located outside of a private room, semi-private room or ward area, thereby replacing the individual bedside processing units for each patient as described previously. Instead, the bedside data concentrator is connected through an appropriate interface and cabling to the local area network, where the data gathered from the clinical devices is then available for processing by the processing unit and displaying the processed data at the various monitoring stations, such as either in the pharmacy or at the nurse station of the institution.

In still an alternative embodiment, the file server and monitoring stations are connected using appropriate interfaces and ethernet cabling to an RF data concentrator. In this embodiment, the clinical devices and (bar)code reader at the bedside are connected to an RF transmitter/receiver, which transmits the information gathered from the clinical devices and the reader to the RF data concentrator attached to the local area network. Thus, expensive cabling is not required to connect every patient treatment area by means of separate bedside processing units or a bedside data concentrator.
In further particular embodiments, all of the information can be transmitted throughout the local area network by way of radio transmission rather than by using costly network cabling.

Accordingly, it will be clear from the disclosure herein that the present invention provides methods combining all of the various services and units of a health care institution into an integrated automated system for accurately efficiently and correctly delivering therapeutic and other drugs to the patient. These methods prevent administering an inappropriate medication to a patient by checking the medication against a database of known allergic reactions and/or side-effects of the drug against the patent's medical history. These methods should also provide doctors, nurses and other care-givers with updated patient information at the bedside, notify the institution's pharmacy when an additional drug is required, or when a scheduled treatment is running behind schedule, and automatically update the institution's accounting database each time a medication or other care is given.

In general, it will be appreciated by the person skilled in the art that the monitoring systems and methods of the present invention will typically be used in a healthcare environment, more particularly in hospitals, clinics and other healthcare institutions. Typical persons handling the systems of the present inventions thus include but are not limited to medical staff, such as nurses, doctors or other medical personnel, such as medical assistants.

However, it will also be understood that the monitoring systems of the present invention may well be applied in a home environment, and thus may be applied by a medical responsible providing domiciliary or home care, i.e. care to patients in their own homes who require administration of medical fluid. Moreover, the monitoring systems of the present invention may also be operated by the patient receiving the medical fluid.

While the invention is susceptible to various modifications, and alternative forms, specific examples thereof have been shown in the drawings and are herein described in detail.

### EXAMPLES

### 1. Illustration of a monitoring system according to the invention in Figure 1

Figure 1 shows a particular embodiment of a monitoring system in accordance with the present invention. Generally, an infusion delivery device is shown, comprising a medical liquid container (4) having a data storage medium (5), such as a machine-readable label, attached thereto. The monitoring system of the invention (1) includes an identification unit (2), such as for example an electronic reader, for acquiring data from the data storage medium and/or a local computer.

Generally, the infusion delivery device can be any device that is used to provide a medical product, such as a vaccine, pharmaceutical, a nutrient (additive), or any other therapeutic or diagnostic agent, to an individual patient. The mode of administration using the delivery device may include for example intravenous, oral or nasal administration into the human body. The delivery device in principle can be a syringe or other injection device, that contains a medical product, such as a vaccine, pharmaceutical, a nutrient (additive), or any other therapeutic or diagnostic agent. More particularly, the delivery infusion device is a device to which a medical fluid container can be attached which in turn may be connected by a hollow tube, optionally connected to a needle guard device that may be attached to a syringe or medical cartridge. In particular embodiments, the medical fluid container may be a vial or bottle, or bag for a liquid medical product.

The data storage medium (5) is attached to the medical liquid container (4), and in particular such that the data storage medium is substantially inseparable from the medical liquid container before administration to a patient.

In particular embodiments, the data storage medium (5) is a read/write machine-readable communications device, such as an electronic recording device, e.g. a "smart" chip or "smart" label, a magnetic strip, and the like. Alternatively, the data storage medium (5) may be a read-only device, such as a barcode strip. In the embodiment illustrated in Figure 1, the data storage medium (5) is attached to the medical liquid container (4) such that it does not interfere substantially with use of the container but may be accessed by the identification unit (2) of the monitoring systems of the invention, e.g. a reader.

The data storage medium (5) comprises information related to the medical liquid product within the medical liquid container (4). For example, the data storage medium (6) may include a product identifier uniquely associated with the medical product. In addition, the data storage medium may provide other information, such as dosage data, e.g. identifying an amount and/or concentration of the medical product within the medical liquid container (4) or identifying a required frequency of administration, and/or an intended patient identifier identifying a patient intended to receive the medical product. Other optional information that may be linked to the product identifier includes product manufacturing date, administration requirements, instructions for use, and/or product warnings, such as possible allergic reactions or adverse interactions of the product with other medical products.

A patient data storage medium (not shown) is associated with an individual patient and provides a patient identifier. For example, the patient identifier may be linked to a patient's name, social security number, or other unique identifier, e.g. associated with a patient upon admittance to a medical provider facility.

Returning to Figure 1, the identification unit (2) of the monitoring systems of the invention, e.g. a reader, is an electronic unit or device configured for uploading information from a data storage medium. In the configuration illustrated in Figure 1, it is configured for transferring this uploaded information elsewhere, e.g. to the processing unit and/or into the local network, or to a location remote from the provider facility, such as a database server (6). Generally, the identification unit (2) may include a sensor, a display, and optionally a memory. Optionally, the identification unit (2) may also include one or more other user interfaces, such as a control keys, or even a keypad, a mouse, and the like. In the illustrated embodiment, the identification unit (2) is coupled via a processing unit (3) to a database server (6) via a wireless or physical communication interface (7), which database server may optionally be connected to a graphical user interface (10) (gui), comprising for example one or more terminals, smart phones, ipads, etc.. The interface (7) may be a cable directly coupling the identification unit to the local network (9) or may be a wireless communications device, such as a short-range transmitter/receiver, a modem, and the like. Alternatively, the identification unit (2) may be a stand-alone device, e.g. with an optionally internal processor and memory. The identification unit (2) is generally configured for uploading information from the data storage medium (5). For example, the infusion delivery device and the identification unit (2) may be placed in close proximity to one another in order to upload the product identifier and/or other information stored in the data storage medium (5). In addition, the identification unit (2) may also be configured for uploading the patient identifier or other information from the patient data storage medium (not shown) associated with an individual patient. Alternatively, the identification unit (2) may include the patient identifier in its internal memory.

The weight monitoring unit (8) measures the weight of the medical liquid container (4) of the medical infusion delivery device when liquid is dispensed therefrom. The weight monitoring unit consists of a device, from which the medical liquid container is suspended. The weight monitoring unit (8) measures the weight of the medical liquid container during the infusion process, i.e. during emptying of the container. In particular embodiments, the weight of the medical liquid container is periodically measured and sent to the processing unit (3), in order to detect when the container is nearly empty, when there is a disruption in fluid-flow, or when a preselected amount of fluid loss has been reached. In particular embodiments, the weight monitoring unit measures absolute weight values of the medical infusion container over time. In other particular embodiments, the weight monitoring unit measures the weight decrease of the medical container and thus measures relative differences between the absolute weights of the medical container over time.

The processing unit (3) may be configured for performing several tasks. With further reference to Figure 1, the processor comprised in the processing unit (3) is configured for processing data acquired from the identification unit (2) and the weight monitoring unit (8). The processing unit may for example be configured for comparing the initial weight value of the medical liquid container with the measured weight values obtained from the weight monitoring unit. In this way, it can be detected by means of the processing unit when the medical container is nearly empty.

The processing unit (3) may further be configured to compare a patient identifier associated with a particular patient (stored in a patient data storage medium or alternatively in the memory of the identification and/or processing unit) with an intended patient identifier acquired from a data storage medium (5) attached to a medical liquid container (4) to determine whether the patient is intended to receive the medical product in the medical liquid container (4).

In addition, the processor comprised in the processing unit (3) may use a product identifier acquired from the data storage medium (6) to request or otherwise obtain additional information regarding the medical product within the medical liquid container (5), e.g. from elsewhere in the local network or from a computer or other remote device (not shown).

The monitoring systems of the present invention illustrated in Figure 1 include a local network (9) located at a provider location. The local network (9) includes a database server (6), which may optionally be connected to a graphical user interface (10) (gui), comprising for example one or more terminals, smart phones, ipads, etc.. In addition, the system may include one or more computers located remotely from the local network (9). The local network (9) and the database server (6) may have access and/or be connected to a communications network (11), such as the internet. In addition, the monitoring systems may include one or more manufacturer computers, which may have access to and/or be connected to the network (9). Preferably, the local network (9) and the manufacturer computer may communicate with a computer via the communications network, but not directly with one another. The database server (6) generally includes a processor, an interface for communicating via the communications network, and memory including patient data files, as described further herein in detail. The identification unit (2) may be coupled to the database server (6), and further optionally to a graphical user interface (10) (GUI) by any conventional device, e.g., directly by a cable, by a wireless transmitter/receiver, and/or by a local area network interface. The database server (6) may include multiple discrete components or computer devices together or distributed about the local network (9) in addition to or instead of the processor, interface, and memory. In addition, the database server (6) may include patient data files including patient identifiers identifying individual patients, and other information related to the individual patients. For example, the patient data files may include patients' names, addresses, personal information, e.g., race, sex, age, etc., medical history, allergies, scheduled medications or prescriptions, and the like.

A manufacturer may download product data into a data storage medium (5) attached to every medical liquid container (4). Because of limited capacity of the data storage medium (5), it may be desirable to minimize the information stored thereon, and, instead, provide only an identifier that may be used to acquire product data associated with the medical product therein. Accordingly, a product identifier may be stored on the data storage medium (5). The manufacturer may store additional product data associated with the product identifier within its internal computer systems.

Before administering the medical product in a medical liquid container (5) to an individual patient, the product identifier stored in the data storage medium (6) may be uploaded into the local network (9) at the provider location. For example, the identification unit (2) may be used to acquire the product identifier, as described above, and the product identifier may be transferred to the processing unit (3) and optionally to the database server (6) coupled to the local network (9). The database server (6) may query the medical product database, submitting the product identifier and acquiring product data associated with the product identifier. Alternatively, the product data may be transferred to the database server (6) when the medical liquid container (5) is received from the manufacturer. Thus, the database server (6) may already include a local database including product data that may be accessed by providing an associated product identifier.

Using the product identifier, one or more confirmations may be completed at the provider location, which may be performed by the identification unit (2), the database server (6), and/or other components of the local network (9), before administering the medical product. For example, the identification unit (2) may acquire a patient identifier associated with the individual patient, as described above, which may be used to identify a patient data file associated with the individual patient. The product data may be used to identify the medical product in the medical liquid container (5), which may then be correlated with data in the individual patient's data file. For example, the database server (6) may confirm that the individual patient is not allergic to the medical product identified by the product identifier. In addition, the database server (6) may access any other medical products being received by the individual patient, and consult a drug interaction database to confirm that there are no adverse reactions with the medical product being administered. Further, the database server (6) may consult a history in the patient data file to confirm that dosage of the medical product is correct or that administration is not being duplicated (i.e. that the medical product is being administered within a desired frequency). If any of these confirmations indicate that administration should not proceed, the identification unit (2) and/or processing unit (3) may provide an output to discontinue administration or to correct administration, e.g. to correct dosage, and the like. In particular embodiments, the identification unit (2) and/or the processing unit (3) may generate a warning signal when for example the information concerning the identity of the patient to be treated is incompatible with the information concerning the content of the medical liquid container.

If the results of any and all confirmations performed are positive, the medical product may then be administered to an individual patient. Upon reading the data storage medium (6), and confirming that administration should proceed, data may be added to the patient data file. For example, administration time data identifying the time of administering the medical product to the individual patient, and/or product data, such as dosage data or other information related to the medical product administered to the individual patient may be added to the patient data file for the individual patient present on the database server (6). In addition, information identifying the individual provider administering the medical product may also be stored in the patient data file in the database server (6), thereby identifying responsible individuals. Data related to consumption of the medical product may be transferred to a system or individual responsible for inventorying medical products at the provider location. Furthermore, data identifying the individual patient and the medical product administered may be transferred to a system or individual responsible for billing and/or other administrative tasks.

### 2. Description of a specific embodiment of the systems of the invention and its practical use

The following describes one specific embodiment of a system for monitoring medical liquid dispensing devices according to the present invention.

In this embodiment, the system according to the invention is provided in a medical care centre, and is used by a medical responsible to monitor a medical liquid dispensing device of a patient. In this embodiment, the system comprises a barcode scanner, which is connected to the IV stand. The IV stand comprises a weight monitoring unit with a hook for hanging an IV bag thereon, which comprises an S-beam load cell attached to a specific signal amplifier with RS-232 communication. The barcode scanner comprises a button which is connected to the microprocessor and the barcode scanner. The system further comprises a database server connected to the microprocessor and the barcode scanner through a TCP/IP connection and a computer in the medical staff unit. The microprocessor is further connected to a means for providing an audible and/or visible warning signal on the weight monitoring unit on or in the vicinity of the hook.

When the patient is to be treated with an IV bag, the barcode present on the IV bag is scanned by the medical responsible using the barcode scanner. The scanning is performed by pressing the button. The barcode is transmitted over Ethernet to the database server via the TCP/IP connection according to the HTTP protocol in form of a HTTP request with the necessary data. The database server receives the data including the barcode information and the IV stand ID. Information regarding which IV stand belongs to which patient is stored in the database (this can be automated afterwards when the patient has a personal barcode). Compatibility of the scanned information and the stored information is checked, i.e. by evaluating whether the scanned IV bag barcode corresponds to the correct patient identity. The HTTP request sends the signal "true" to the microprocessor when the patient is allowed to receive the respective IV bag or sends the signal "false" when the IV bag is not in the list of allowed products for that particular patient.

When the microprocessor at the IV stand receives a "false" signal from the database server, an (audible and/or visual) alarm is triggered. Alternatively, when the microprocessor at the IV stand receives a "true" signal, the indication light of the weight monitoring hook that has to be used starts flashing. This is an indication for the medical responsible allowing him to hang the IV bag on the weight monitoring hook. After a certain time period, when the weight monitoring unit has detected a stable added weight, the indication light is lit continuously.

From now on the microprocessor starts receiving weight values of the IV bag measured by the weight monitoring sensor by use of a serial data transmission protocol (RS-232). At a configurable interval, the microprocessor sends a HTTP request over TCP/IP to the DB server with the Stand ID, Hook ID, Barcode and last measured weight value in the URL. The database server stores all of the data in a MYSQL database.
At this point, the database server can calculate the weight loss as a function of time. This value is compared to a set threshold value for the according IV bag (also stored in the database). The data can be accessed by the medical staff in the nurse unit through a web interface (HTML + PHP). This webpage can optionally also be viewed by IPAD's smart-phones or other devices that have internet browser capabilities.

The data are calculated for each IV bag that is in use by any patient in the hospital. This way the graphical interface (Website) can be used to provide an overview of the status of all IV bags that are in use.

Audible and/or visual alarms can be generated at the medical staff unit in order to get the attention when an alarm value is reached. This event is triggered by a web application that checks the database for alarm values. These alarm conditions are continuously checked when the web application sends a request to the database server. When a check-up tour is performed by the medical responsible, the status of the IV bags on the tour can be taken into account (planning such a status check-up can be automated using suitable software).

When an empty IV bag is detached by the medical responsible, no extra weight is measured by the weight sensor and a "clear hook" signal is sent by the microprocessor to the database server. The microprocessor subsequently stops monitoring this particular hook and the hook's indication light is switched off. The hook is now available to be used for a new IV bag.

### 3. Use of the methods of the present invention for monitoring the dispensing of the infusion liquid - use of bedside (i.e. local) processing units

In the presently illustrated embodiment, information is collected from each patient into the monitoring system described herein. The patient wears an identification device that includes a barcode that can be read by a barcode reader connected to a bedside processing unit, i.e. in the direct vicinity of the dispensing device. Medication to be administered to the patient in the course of the patient's treatment is identified with a label that is printed by a barcode printer in the pharmacy or by the manufacturer's supplied barcodes on unit dose packaging. When the medication is administered to the patient by a care-giver, nurse, or medical assistant, the barcode reader is used, which is connected to the bedside processing unit to read the barcode on the patient's identification device and the barcode on the label identifying the medication to be dispensed. The processing unit of the monitoring systems of the invention compares the patient's identity with the medication and verifies that it is the correct medication for the patient. Additionally, the caregiver may also have an identification device that bears a barcode with the caregiver's name and other information. Using the barcode reader, the care giver's identity can thus be stored in the database and linked to the treatment given to the patient to ensure complete and accurate tracking of all treatment given to the patient.

The bedside processing unit is used by a nurse, physician or technician to access a variety of institutional databases to display a variety of information about a particular patient. This information can include a patient medication administration record that is derived from the patient's medication profile maintained by the hospital's pharmacy information system. The bedside processing unit also allows remote access to a patient's records stored by the file server to display medication history for the patient. This medication history includes a listing of all drug or other treatments including past, present and future deliveries to the patient. Additionally, access to administration records of the hospital's administration system is available through the network of computers. Each bedside processing unit is connected through an appropriate interface to a variety of peripheral equipment.

It is clear from the above, that the monitoring systems of the present invention facilitate updating patient records and ensure that medical products are administered in a timely manner and are not repeated too frequently.

## Claims

1. A monitoring system for monitoring the dispensing of a liquid from a medical liquid dispensing device for administration into a patient comprising:
- an identification unit for identifying a medical liquid container via a data storage medium comprising a product identifier,
- a weight monitoring unit for measuring the weight of said medical liquid container when liquid is dispensed therefrom and
- a processing unit for processing and/or storing data acquired from said identification unit and said weight monitoring unit, which processing unit is interconnected with said identification unit and said weight monitoring unit via a wireless or physical communications interface;
wherein said processing unit is configured to
- compare information concerning the identity of said patient, uploaded as a patient identifier, with information obtained from said product identifier; and to compare said weight measured by said weight monitoring unit with an expected weight value based on the flow of said liquid dispensing device and to generate a warning signal when said measured weight is above or below said expected weight value by means of said processing unit.

2. The monitoring system according to claim 1, wherein said flow is determined by comparing the weight change of the medical liquid container, determined by comparing subsequent weight data transmitted by the weight monitoring unit, to the weight of said liquid.

3. The monitoring system according to claim 1 or 2, wherein said processing unit comprises a processor configured for processing data acquired from said identification unit and said weight monitoring unit.

4. The monitoring system according to claims 1 to 3, wherein said data acquired from said identification unit and said weight monitoring unit include information concerning the identity of said medical liquid container and optionally concerning the identity of the patient to be treated with said medical liquid dispensing device.

5. The monitoring system according to claim 4, wherein said information concerning the identity of said medical liquid container at least includes information concerning the weight of said medical liquid container and optionally concerning the content of said medical liquid container.

6. The monitoring system according to any of claims 1 to 5, wherein said processing unit is further connected to an external database server system.

7. The monitoring system according to any of claims 1 to 6, wherein said weight monitoring unit comprises at least one electronic weight sensor.

8. The monitoring system according to any of claims 1 to 7, wherein said identification unit comprises a reader and wherein said data storage medium comprises a machine-readable label.

9. A method for monitoring the dispensing of a liquid from a medical liquid dispensing device for administration into a patient comprising the steps of:
- identifying by means of an identification unit a medical liquid container via a data storage medium comprising a product identifier,
- measuring the weight of said medical liquid container when liquid is dispensed therefrom by means of a weight monitoring unit,
- processing and/or storing data acquired from said identification unit and said weight monitoring unit by means of a processing unit, including comparing the patient information uploaded as patient identifier in said processing unit with information concerning said medical liquid container obtained from said product identifier; and
- comparing said weight measured by said weight monitoring unit with an expected weight value based on the flow of said liquid dispensing device and generating a warning signal when said measured weight is above or below said expected weight value by means of said processing unit.

10. The method according to claim 9, wherein said flow is determined by comparing the weight change of the medical liquid container, determined by comparing subsequent weight data transmitted by the weight monitoring unit, to the weight of said liquid.

11. The method according to claim 9 or 10, wherein said step of identifying by means of an identification unit a medical liquid container via a data storage medium at least comprises obtaining information from said processing unit concerning the weight of said medical liquid container and optionally concerning the content of said medical liquid container.

12. The method according to any one of claims 9 to 11, wherein said step of measuring the weight of a medical liquid container at least comprises measuring the weight decrease of said medical liquid container when liquid is dispensed therefrom by means of a weight monitoring unit.

13. The method according to any of claims 9 to 12, wherein said method further comprises the step of comparing said weight measured by said weight monitoring unit to a predetermined threshold value and optionally generating a warning signal when said measured weight corresponds to or is below said threshold value by means of said processing unit.

14. The method according to claim 13, wherein said predetermined threshold value is determined based on said information obtained from said processing unit concerning the weight of said medical liquid container.

15. The method according to claims 9 to 14, wherein said method comprises the step of
- generating a warning signal when a defect in the flow mechanism of said medical liquid container is detected by said processing unit based on said weight measured by said weight monitoring unit; and/or
- generating a warning signal by means of said processing unit when said information concerning the identity of the patient to be treated is incompatible with said information concerning the content of the medical liquid container.

## Patentansprüche

1. Überwachungssystem zur Überwachung der Abgabe einer Flüssigkeit aus einer medizinischen Flüssigkeitsabgabevorrichtung zur Verabreichung in einen Patienten, die Folgendes umfasst:
- eine Identifikationseinheit zum Identifizieren eines medizinischen Flüssigkeitsbehälters über ein Datenspeichermedium, das eine Produktkennung aufweist,
- eine Gewichtsüberwachungseinheit zum Messen des Gewichts des medizinischen Flüssigkeitsbehälters, wenn Flüssigkeit daraus abgegeben wird, und
- eine Verarbeitungseinheit zum Verarbeiten und/oder Speichern von Daten, die von der Identifikationseinheit und der Gewichtsüberwachungseinheit erfasst werden, wobei die Verarbeitungseinheit mit der Identifikationseinheit und der Gewichtsüberwachungseinheit über eine drahtlose oder physikalische Kommunikationsschnittstelle verbunden ist;
wobei die Verarbeitungseinheit konfiguriert ist
- Informationen, die die als eine Patientenkennung hochgeladene Identität des Patienten betreffen, mit Informationen zu vergleichen, die aus der Produktkennung erhalten werden; und das durch die Gewichtsüberwachungseinheit gemessene Gewicht mit einem erwarteten Gewichtswert zu vergleichen, der auf dem Fluss der Flüssigkeitsabgabevorrichtung beruht, und mittels der Verarbeitungseinheit ein Warnsignal zu erzeugen, wenn das gemessene Gewicht über oder unter dem erwarteten Gewichtswert liegt.

2. Überwachungssystem nach Anspruch 1, wobei der Fluss durch Vergleichen der Gewichtsänderung des medizinischen Flüssigkeitsbehälters, die durch Vergleichen aufeinanderfolgender Gewichtsdaten festgestellt wird, die durch die Gewichtsüberwachungseinheit übertragen werden, mit dem Gewicht der Flüssigkeit festgestellt wird.

3. Überwachungssystem nach Anspruch 1 oder 2, wobei die Verarbeitungseinheit einen Prozessor aufweist, der zum Verarbeiten von Daten konfiguriert ist, die von der Identifikationseinheit und der Gewichtsüberwachungseinheit erfasst werden.

4. Überwachungssystem nach Anspruch 1 bis 3, wobei die von der Identifikationseinheit und der Gewichtsüberwachungseinheit erfassten Daten Informationen umfassen, die die Identität des medizinischen Flüssigkeitsbehälters betreffen und optional die Identität des Patienten betreffen, der mit der medizinischen Flüssigkeitsabgabevorrichtung behandelt werden soll.

5. Überwachungssystem nach Anspruch 4, wobei die Informationen, die die Identität des medizinischen Flüssigkeitsbehälters betreffen, mindestens Informationen umfassen, die das Gewicht des medizinischen Flüssigkeitsbehälters betreffen und optional den Inhalt des medizinischen Flüssigkeitsbehälters betreffen.

6. Überwachungssystem nach einem der Ansprüche 1 bis 5, wobei die Verarbeitungseinheit ferner mit einem externen Datenbankserversystem verbunden ist.

7. Überwachungssystem nach einem der Ansprüche 1 bis 6, wobei die Gewichtsüberwachungseinheit mindestens einen elektronischen Gewichtssensor aufweist.

8. Überwachungssystem nach einem der Ansprüche 1 bis 7, wobei die Identifikationseinheit ein Lesegerät aufweist und wobei das Datenspeichermedium ein maschinenlesbares Etikett aufweist.

9. Verfahren zum Überwachen der Abgabe einer Flüssigkeit aus einer medizinischen Flüssigkeitsabgabevorrichtung zur Verabreichung in einen Patienten, das die folgenden Schritte aufweist:
- Identifizieren mittels einer Identifikationseinheit eines medizinischen Flüssigkeitsbehälters über ein Datenspeichermedium, das eine Produktkennung aufweist,
- Messen des Gewichts des medizinischen Flüssigkeitsbehälters, wenn daraus Flüssigkeit abgegeben wird, mittels einer Gewichtsüberwachungseinheit,
- Verarbeiten und/oder Speichern von Daten, die von der Identifikationseinheit und der Gewichtsüberwachungseinheit erfasst werden, mittels einer Verarbeitungseinheit, einschließlich Vergleichen der als Patientenkennung in die Verarbeitungseinheit hochgeladenen Patienteninformationen mit Informationen, die den medizinischen Flüssigkeitsbehälter betreffen, die aus der Produktkennung erhalten werden; und
- Vergleichen des durch die Gewichtsüberwachungseinheit gemessenen Gewichts mit einem erwarteten Gewichtswert, der auf dem Fluss der Flüssigkeitsabgabevorrichtung beruht, und Erzeugen eines Warnsignals, wenn das gemessene Gewicht über oder unter dem erwarteten Gewichtswert liegt, mittels der Verarbeitungseinheit.

10. Verfahren nach Anspruch 9, wobei der Fluss durch Vergleichen der Gewichtsänderung des medizinischen Flüssigkeitsbehälters, die durch Vergleichen aufeinanderfolgender Gewichtsdaten festgestellt wird, die durch die Gewichtsüberwachungseinheit übertragen werden, mit dem Gewicht der Flüssigkeit festgestellt wird.

11. Verfahren nach Anspruch 9 oder 10, wobei der Schritt des Identifizierens mittels einer Identifikationseinheit eines medizinischen Flüssigkeitsbehälter über ein Datenspeichermedium mindestens das Erhalten von Informationen aus der Verarbeitungseinheit aufweist, die das Gewicht des medizinischen Flüssigkeitsbehälters betreffen und optional den Inhalt des medizinischen Flüssigkeitsbehälters betreffen.

12. Verfahren nach einem der Ansprüche 9 bis 11, wobei der Schritt des Messens des Gewichts eines medizinischen Flüssigkeitsbehälters mindestens das Messen der Gewichtsabnahme des medizinischen Flüssigkeitsbehälters, wenn daraus Flüssigkeit abgegeben wird, mittels einer Gewichtsüberwachungseinheit aufweist.

13. Verfahren nach einem der Ansprüche 9 bis 12, wobei das Verfahren ferner den Schritt des Vergleichens des durch die Gewichtsüberwachungseinheit gemessenen Gewichts mit einem vorgegebenen Schwellenwert und optional das Erzeugen eines Warnsignals, wenn das gemessene Gewicht dem Schwellenwert entspricht oder darunter liegt, mittels der Verarbeitungseinheit aufweist.

14. Verfahren nach Anspruch 13, wobei der vorgegebene Schwellenwert beruhend auf den Informationen festgestellt wird, die aus der Verarbeitungseinheit erhalten werden, die das Gewicht des medizinischen Flüssigkeitsbehälters betreffen.

15. Verfahren nach den Ansprüchen 9 bis 14, wobei das Verfahren den folgenden Schritt aufweist
- Erzeugen eines Warnsignals, wenn durch die Verarbeitungseinheit beruhend auf dem durch die Gewichtsüberwachungseinheit gemessenen Gewicht ein Defekt im Flussmechanismus des medizinischen Flüssigkeitsbehälters ermittelt wird; und/oder
- Erzeugen eines Warnsignals mittels der Verarbeitungseinheit, wenn die Informationen, die die Identität des zu behandelnden Patienten betreffen, mit den Informationen inkompatibel sind, die den Inhalt des medizinischen Flüssigkeitsbehälters betreffen.

## Revendications

1. Système de surveillance destiné à surveiller la distribution d'un liquide provenant d'un dispositif de distribution de liquide médical pour une administration à un patient, comprenant :
- une unité d'identification pour identifier un conteneur de liquide médical par l'intermédiaire d'un support de stockage de données comprenant un identifiant de produit,
- une unité de surveillance de poids pour mesurer le poids dudit conteneur de liquide médical lorsque du liquide est distribué à partir de celui-ci, et
- une unité de traitement pour traiter et/ou stocker des données acquises par ladite unité d'identification et ladite unité de surveillance de poids, laquelle unité de traitement est interconnectée avec ladite unité d'identification et ladite unité de surveillance de poids par l'intermédiaire d'une interface de communication sans fil ou physique ;
où ladite unité de traitement est configurée pour :
- comparer des informations concernant l'identité dudit patient, téléchargées comme identifiant du patient, à des informations obtenues à partir dudit identifiant de produit ; et comparer ledit poids mesuré par ladite unité de surveillance de poids à une valeur de poids attendue sur la base du débit dudit dispositif de distribution de liquide, et générer un signal d'avertissement lorsque ledit poids mesuré est au-dessus ou en-dessous de ladite valeur de poids attendue au moyen de ladite unité de traitement.

2. Système de surveillance selon la revendication 1, dans lequel ledit débit est déterminé en comparant le changement de poids du conteneur de liquide médical, déterminé en comparant des données de poids subséquentes transmises par l'unité de surveillance de poids, au poids dudit liquide.

3. Système de surveillance selon la revendication 1 ou la revendication 2, dans lequel ladite unité de traitement comprend un processeur configuré pour traiter des données acquises auprès de ladite unité d'identification et de ladite unité de surveillance de poids.

4. Système de surveillance selon les revendications 1 à 3, dans lequel lesdites données acquises auprès de ladite unité d'identification et de ladite unité de surveillance de poids comprennent des informations concernant l'identité dudit conteneur de liquide médical et, optionnellement, concernant l'identité du patient à traiter avec ledit dispositif de distribution de liquide médical.

5. Système de surveillance selon la revendication 4, dans lequel lesdites informations concernant l'identité dudit conteneur de liquide médical comprennent au moins des informations concernant le poids dudit conteneur de liquide médical et, optionnellement, concernant le contenu dudit conteneur de liquide médical.

6. Système de surveillance selon l'une quelconque des revendications 1 à 5, dans lequel ladite unité de traitement est en outre connectée à un système de serveur de base de données externe.

7. Système de surveillance selon l'une quelconque des revendications 1 à 6, dans lequel ladite unité de surveillance de poids comprend au moins un capteur de pesage électronique.

8. Système de surveillance selon l'une quelconque des revendications 1 à 7, dans lequel ladite unité d'identification comprend un lecteur, et dans lequel ledit support de stockage de données comprend une étiquette lisible par une machine.

9. Procédé pour surveiller la distribution d'un liquide depuis un dispositif de distribution de liquide médical pour une administration à un patient, comprenant les étapes suivantes :
- identifier, au moyen d'une unité d'identification, un conteneur de liquide médical par l'intermédiaire d'un support de stockage de données comprenant un identifiant de produit,
- mesurer le poids dudit conteneur de liquide médical lorsque du liquide est distribué depuis celui-ci au moyen d'une unité de surveillance de poids,
- traiter et/ou stocker des données acquises par ladite unité d'identification et ladite unité de surveillance de poids au moyen d'une unité de traitement, comprenant de comparer les informations du patient téléchargées comme identifiant du patient dans ladite unité de traitement à des informations concernant ledit conteneur de liquide médical obtenues par ledit identifiant de produit ; et
- comparer ledit poids mesuré par ladite unité de surveillance de poids à une valeur de poids attendue sur la base du débit dudit dispositif de distribution de liquide, et générer un signal d'avertissement lorsque ledit poids mesuré est au-dessus ou en-dessous de ladite valeur de poids attendue au moyen de ladite unité de traitement.

10. Procédé selon la revendication 9, dans lequel ledit débit est déterminé en comparant le changement de poids du conteneur de liquide médical, déterminé en comparant des données de poids subséquentes transmises par l'unité de surveillance de poids, au poids dudit liquide.

11. Procédé selon la revendication 9 ou la revendication 10, dans lequel ladite étape d'identification au moyen d'une unité d'identification d'un conteneur de liquide médical par l'intermédiaire d'un support de stockage de données comprend au moins d'obtenir des informations depuis ladite unité de traitement concernant le poids dudit conteneur de liquide médical et, optionnellement, concernant le contenu dudit conteneur de liquide médical.

12. Procédé selon l'une quelconque des revendications 9 à 11, dans lequel ladite étape de mesure du poids d'un conteneur de liquide médical comprend au moins de mesurer la diminution de poids dudit conteneur de liquide médical, lorsque du liquide est distribué à partir de celui-ci, au moyen d'une unité de surveillance de poids.

13. Procédé selon l'une quelconque des revendications 9 à 12, dans lequel ledit procédé comprend en outre l'étape comprenant de comparer ledit poids mesuré par ladite unité de surveillance de poids à une valeur seuil prédéterminée et, optionnellement, de générer, au moyen de ladite unité de traitement, un signal d'avertissement lorsque ledit poids mesuré correspond, ou est inférieur, à ladite valeur seuil.

14. Procédé selon la revendication 13, dans lequel ladite valeur seuil prédéterminée est déterminée sur la base desdites informations obtenues depuis ladite unité de traitement concernant le poids dudit conteneur de liquide médical.

15. Procédé selon les revendications 9 à 14, où ledit procédé comprend les étapes suivantes :
- générer un signal d'avertissement lorsqu'une anomalie dans le mécanisme d'écoulement dudit conteneur de liquide médical est détectée par ladite unité de traitement sur la base dudit poids mesuré par ladite unité de surveillance de poids ; et/ou
- générer un signal d'avertissement au moyen de ladite unité de traitement lorsque lesdites informations concernant l'identité du patient à traiter sont incompatibles avec lesdites informations concernant le contenu du conteneur de liquide médical.
